(19) Europäisches Patentamt
European Patent Office
Office européen des brevets

(11) Numéro de publication: **0 540 400 B1**

(12) **FASCICULE DE BREVET EUROPEEN**

(45) Date de publication du fascicule du brevet: **23.08.95**

(51) Int. Cl.[6]: **C07D 401/14**, C07D 215/12, A61K 31/47

(21) Numéro de dépôt: **92402879.8**

(22) Date de dépôt: **22.10.92**

Le dossier contient des informations techniques présentées postérieurement au dépôt de la demande et ne figurant pas dans le présent fascicule.

(54) **Dérivés de quinoléine, utile comme angiotensine II antagonistes.**

(30) Priorité: **28.10.91 FR 9113240**

(43) Date de publication de la demande: **05.05.93 Bulletin 93/18**

(45) Mention de la délivrance du brevet: **23.08.95 Bulletin 95/34**

(84) Etats contractants désignés: **AT BE CH DE DK ES FR GB GR IE IT LI LU MC NL PT SE**

(56) Documents cités:

**JOURNAL OF MEDICINAL CHEMISTRY. vol. 33, 1990, WASHINGTON US pages 1330 - 1336 D.J.CARINI ET AL. 'Nonpeptide angiotensin II antagonists: N-(benzyloxy)benzyl)imidazoles and related compounds as potent anihypertensives.'**

(73) Titulaire: **SYNTHELABO**
**22, Avenue Galilée**
**F-92350 Le Plessis Robinson (FR)**

(72) Inventeur: **Cremer, Gérard**
**35, rue de Sables**
**F-91420 Morangis (FR)**
Inventeur: **Goberville, Pascale**
**105, rue Ledru-Rollin**
**F-94100 Saint-Maur (FR)**
Inventeur: **Muller, Jean-Claude**
**4, avenue de l'Espérance**
**F-91390 Morsang Sur Orge (FR)**

(74) Mandataire: **Thouret-Lemaitre, Elisabeth et al**
**SYNTHELABO,**
**Service Brevets,**
**B.P. 72**
**F-92352 Le Plessis-Robinson Cédex (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de quinoléine, leur préparation et leur application en thérapeutique.

Les composés de l'invention répondent à la formule générale (I)

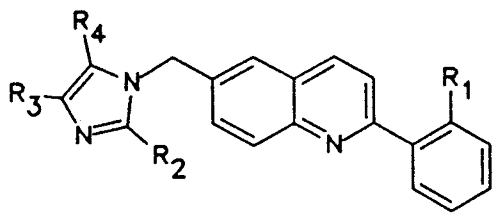

(I)

dans laquelle

$R_1$ représente soit un groupe 1*H*-tétrazol-5-yle, soit un groupe $CO_2H$,

$R_2$ représente soit un groupe $(C_{1-7})$alkyle, soit un groupe $(C_{2-6})$alcényle,

$R_3$ et $R_4$ représentent, chacun indépendamment l'un de l'autre, soit un atome d'hydrogène, soit un atome d'halogène, soit un groupe cyano, soit un groupe $(C_{1-7})$alkyle, soit un groupe $(C_{3-7})$cycloalkyl-$(C_{1-4})$alkyle, soit un groupe aryle, soit un groupe aryl$(C_{1-4})$alkyle, soit un groupe aryl$(C_{2-6})$alcényle, soit un groupe $-(CH_2)_mCOR_5$ dans lequel m = 0 à 4 et $R_5$ représente un atome d'hydrogène, un groupe OH, un groupe $(C_{1-6})$alcoxy ou un groupe $NR_7R_8$, $R_7$ et $R_8$ représentant, chacun indépendamment l'un de l'autre, un atome d'hydrogène ou un groupe $(C_{1-4})$alkyle, soit un groupe $-(CH_2)_n-R_6$ dans lequel n = 1 à 4 et $R_6$ représente un groupe OH, un groupe $(C_{1-6})$alcoxy, un groupe $(C_{1-4})$alcoxy$(C_{1-4})$alcoxy ou un groupe $(C_{3-7})$cycloalkyl$(C_{1-4})$-alcoxy.

Les composés préférés de l'invention sont ceux pour lesquels

$R_1$ représente soit un groupe 1*H*-tétrazol-5-yle, soit un groupe $CO_2H$,

$R_2$ représente un groupe $(C_{1-7})$alkyle,

$R_3$ représente soit un atome d'halogène, soit un groupe $(C_{1-7})$alkyle, soit un groupe aryl$(C_{1-4})$alkyle,

$R_4$ représente soit un groupe $-(CH_2)_m-COR_5$ dans lequel m = 0 à 4 et $R_5$ représente un atome d'hydrogène, un groupe OH, un groupe $(C_{1-6})$alcoxy ou un groupe $NR_7R_8$, $R_7$ et $R_8$ représentant indépendamment l'un de l'autre, un atome d'hydrogène ou un groupe $(C_{1-4})$alkyle, soit un groupe $-(CH_2)_n-R_6$ dans lequel n = 1 à 4 et $R_6$ représente un groupe OH ou un groupe $(C_{1-6})$alcoxy.

Enfin les composés de choix sont ceux pour lesquels

$R_1$ représente un groupe 1*H*-tétrazol-5-yle,

$R_2$ représente un groupe butyle,

$R_3$ représente soit un atome de chlore, soit un groupe éthyle, soit un groupe phénéthyle,

$R_4$ représente un groupe $CH_2OH$, CHO, $CO_2H$, $CO_2CH_3$, $CO_2C_2H_5$, $CH_2OCH_3$.

Les composés de l'invention peuvent se présenter sous forme libre ou sous forme de sels organiques ou inorganiques pharmaceutiquement acceptables.

D.J. Carini décrit dans J. Med. Chem. 33, 1330-36 (1990), des dérivés de *N*-[(benzyloxy)benzyl] imidazoles.

Les composés de l'invention pour lesquels $R_1$ représente un groupe 1*H*-tétrazol-5-yle peuvent être préparés suivant le schéma de l'annexe I.

Dans une première étape, on fait réagir à la température de reflux, la 4-méthylbenzènamine (paratoluidine) avec un benzaldéhyde de formule (II), dans laquelle X représente un atome de brome ou d'iode, en présence d'un catalyseur tel l'acide 4-méthylbenzènesulfonique (acide paratoluènesulfonique ou PTSA), en solution dans du benzène. Après refroidissement, on ajoute de l'acide propiolique et l'on chauffe à la température de reflux de manière à obtenir le composé de formule (III).

Dans une seconde étape, on chauffe un mélange du composé de formule (III) et de cyanure cuivreux dans un solvant tel que la pyridine, de manière à obtenir le 2-(6-méthylquinoléin-2-yl)benzonitrile (IV).

Dans une troisième étape, on fait réagir le 2-(6-méthylquinoléin-2-yl)benzonitrile avec un azidure organométallique tel l'azidure de triméthylétain, ou un azidure métallique tel l'azidure de sodium, de manière à obtenir un composé sur lequel on fait passer un courant d'acide chlorhydrique gazeux, pour obtenir la 6-méthyl-2-[2-(1*H*-tétrazol-5-yl)-phényl]quinoléine (V). La première réaction est conduite dans un solvant tel que le xylène à la température de reflux; la seconde réaction est conduite dans un solvant tel que le mélange toluène/tétrahydrofurane, à la température ambiante.

Dans une quatrième étape, on protège par un groupe protecteur de formule $CR_{13}R_{14}R_{15}$, dans laquelle $R_{13}$, $R_{14}$ et $R_{15}$ représententchacun indépendamment l'un de l'autre un groupe $(C_{1-2})$alkyle ou un groupe aryle, le groupe tétrazole de la 6-méthyl-2-[2-(1*H*-tétrazol-5-yl)phényl]quinoléine (V); dans cette étape, on fait réagir le composé (V) avec un agent protecteur, tel par exemple le chlorure de trityle à la température ambiante dans un solvant tel que le dichlorométhane, en présence d'une base telle que la N-méthylmorpholine ou la triéthylamine et on obtient un composé de formule (VI) dans laquelle $R_{13}$, $R_{14}$ et $R_{15}$ sont tels que définis précédemment. La protection du groupe tétrazole se fait préférentiellement en position 2.

Dans une cinquième étape, on fonctionnalise le groupe méthyle en position 6 de la quinoléine de formule (VI), en y introduisant un groupe partant. Si le groupe partant est un radical bromo, alors on fait réagir un composé de formule (VI) avec du N-bromosuccinimide de manière à obtenir un composé de formule (VII) dans laquelle $CR_{13}R_{14}R_{15}$ est tel que défini précédemment; la réaction est conduite à la température de reflux dans un solvant tel que le tétrachlorure de carbone, en présence d'un initiateur tel que le peroxyde de benzoyle ou l'$\alpha,\alpha'$-azobisisobutyronitrile.

Dans une sixième étape, on fait réagir un composé de formule (VII) avec un imidazole de formule (VIII), dans laquelle $R_2$, $R_3$ et $R_4$ sont tels que définis précédemment, pour donner un dérivé de formule (IX). La réaction est réalisée dans un solvant tel que le diméthylformamide, à une température de 0 °C à 50 °C en présence d'une base telle le carbonate de potassium.

Dans une septième étape, on réalise la déprotection du groupe tétrazolyle de maniére à obtenir un composé de formule (I bis).

Les composés de l'invention pour lesquels $R_1$ représente un groupe $CO_2H$, peuvent être préparés suivant le schéma de l'annexe II.

Dans une première étape, on fait réagir à la température de reflux, la 4-méthylbenzènamine (paratoluidine) avec un benzaldéhyde de formule (II), dans laquelle X représente un atome de brome ou d'iode, en présence d'un catalyseur tel l'acide 4-méthylbenzènesulfonique (acide paratoluènesulfonique ou PTSA), en solution dans du benzène. Après refroidissement, on ajoute de l'acide propiolique et l'on chauffe à la température de reflux de manière à obtenir le composé de formule (III).

Dans une seconde étape, on chauffe un mélange du composé (III) et de cyanure cuivreux dans un solvant tel que la pyridine, de manière à obtenir le 2-(6-méthylquinoléin-2-yl)benzonitrile (IV).

Dans une troisième étape, on fait réagir le 2-(6-méthylquinoléin-2-yl)benzonitrile avec un alcool R-OH (V') où R est un radical $(C_{1-4})$alkyle ramifié ou non, en présence d'un acide, par exemple l'acide sulfurique, pour obtenir une quinoléine de formule (VI') dans laquelle R est tel que défini précédemment.

Dans une quatrième étape, on fonctionnalise le groupe méthyle en position 6 de la quinoléine en y introduisant un groupe partant. Si le groupe partant est un radical bromo, alors on fait réagir la quinoléine de formule (VI') avec du N-bromosuccinimide de manière à obtenir la quinoléine de formule (VII') dans laquelle R est tel que défini précédemment; la réaction est conduite dans un solvant tel que le tétrachlorure de carbone, en présence d'un initiateur tel que le peroxyde de benzoyle ou l'$\alpha,\alpha'$-azobisisobutyronitrile, à la température de reflux.

Dans une cinquième étape, on fait réagir la quinoléine de formule (VII') avec un imidazole de formule (VIII), dans laquelle $R_2$, $R_3$ et $R_4$ sont tels que définis précédemment, pour donner un dérivé de formule (IX'). La réaction est réalisée dans le diméthylformamide, à une température de 0 °C à 50 °C, en présence d'une base telle que l'hydroxyde de potassium ou le carbonate de potassium.

Dans une sixième étape, on hydrolyse la fonction ester de la quinoléine de formule (IX') de manière à obtenir un composé de formule (I ter).

Les composés intermédiaires sont nouveaux et font partie de l'invention. Ils répondent à la formule (X)

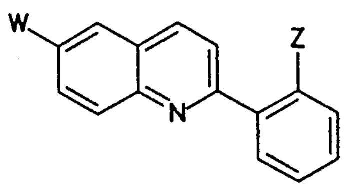

(X)

dans laquelle

W représente soit un groupe méthyle, soit un groupe $-CH_2R_{11}$ dans lequel $R_{11}$ représente un atome de chlore, un atome de brome ou un groupe partant $OR_{12}$, $OR_{12}$ étant un groupe tel qu'un groupe tosyle ou un groupe mésyle,

Z représente soit un atome d'halogène, soit un groupe cyano, soit un groupe 1*H*-tétrazol-5-yle, soit un

groupe 1*H*-tétrazol-5-yle protégé par un groupe protecteur de formule $CR_{13}R_{14}R_{15}$, $R_{13}$, $R_{14}$ et $R_{15}$ représentant chacun indépendamment l'un de l'autre un groupe $(C_{1-2})$alkyle ou un groupe aryle, soit un groupe COOR, R étant un groupe $(C_{1-4})$alkyle ramifié ou non.

Les composés de départ sont disponibles dans le commerce ou décrits dans la littérature ou peuvent être préparés selon des méthodes qui y sont décrites ou qui sont connues de l'homme du métier.

Les exemples suivants illustrent l'invention. Les microanalyses et les spectres IR et RMN confirment la structure des composés obtenus.

Exemple 1

2-butyl-4-chloro-1-[[2-[2-(1*H*-tétrazol-5-yl)phényl]quinoléin-6-yl]méthyl]-1*H*−imidazole-5-carboxaldéhyde, chlorhydrate

1.1. 2-(2-bromophényl)-6-méthylquinoléine

On chauffe à la température de reflux, dans un ballon surmonté d'un Dean-Stark, 50 g (270 mmoles) de 2-bromobenzaldéhyde avec 29,5 g (276 mmoles) de paratoluidine et 0,5 g d'acide paratoluènesulfonique en solution dans un litre de benzène. Lorsque l'élimination d'eau est terminée (environ 5 ml), on ajoute au milieu réactionnel préalablement refroidi vers 50 °C, 8,3 ml (135 mmoles) d'acide propiolique. On note un dégagement important de $CO_2$ et on porte au reflux pendant 3 heures. On contrôle la réaction par chromatographie sur couche mince, dans un mélange de dichlorométhane et d'hexane (70/30). Dans nos conditions expérimentales, il a été nécessaire d'ajouter un excès de 20% d'acide propiolique suivi d'un reflux d'1 heure pour parfaire la réaction. On évapore le solvant sous pression réduite et on purifie le résidu par chromatographie sur colonne de silice, en éluant avec un mélange de dichlorométhane et d'hexane (70/30).

On récupère 22 g du dérivé attendu, sous forme d'un composé cristallisé.

M = 22 g F = 92 °C Rdt = 27%

RMN $^1$H (200 MHz, $CDCl_3$): δ 2,55 (s, 3H), 7,25-7,70 (m, 7H), 8,02-8,15 (m, 2H).

De la même manière, on prépare la 2-(2-iodophényl)-6-méthylquinoléine à partir du 2-iodobenzaldéhyde.

F = 77-77,5 °C

1.2. 2-(6-méthylquinoléin-2-yl)benzonitrile

On chauffe à 160 °C pendant 12 heures, sous argon, un mélange contenant, 15 g (50 mmoles) du composé précédemment obtenu en 1.1 et 5 g (56 mmoles) de cyanure cuivreux dans 60 ml de pyridine. On contrôle la réaction par chromatographie sur couche mince dans du dichlorométhane. On évapore la pyridine sous pression réduite et on reprend le résidu par du dichlorométhane. On lave la phase organique plusieurs fois avec une solution aqueuse d'ammoniaque, jusqu'à ce que la phase aqueuse soit incolore. Après un dernier lavage à l'eau, on sèche la phase organique sur du sulfate de magnésium et on évapore le solvant. On reprend le résidu par de l'éther de pétrole.

M = 9,6 g F = 157 °C Rdt = 78%

1.3. 6-méthyl-2-[2-(1*H*-tétrazol-5-yl)phényl]quinoléine, chlorhydrate

Dans 110 ml de xylène on introduit, 9,6 g (39,29 mmoles) de nitrile obtenu précédemment en 1.2 et 14,96 g (72,7 mmoles) d'azidure de triméthylétain. On porte ce mélange au reflux pendant 15 heures. Après refroidissement, on filtre le solide et on le met en suspension dans 115 ml de toluène et 7 ml de tétrahydrofurane. On soumet le mélange, refroidi par un bain de glace, à un barbotage de gaz chlorhydrique pendant 2 heures. On récupère la fraction insoluble par filtration puis on la lave avec du toluène et avec de l'eau. M = 13 g

1.4. 6-méthyl-2-[2-[2-(triphénylméthyl)-1*H*-tétrazol-5-yl]phényl]quinoléine

Dans un litre de dichlorométhane et à température ambiante, on ajoute, 80,5 g (0,219 mole) du composé obtenu précédemment en 1.3, 60 ml (0,547 mole) de N-méthyl morpholine et 73,1 g (0,262 mole) de chlorure de trityle. On agite la solution pendant une nuit, on reprend par de l'eau, on lave la phase organique deux fois par de l'eau puis on sèche. On évapore le solvant et on cristallise le résidu dans un

minimum d'éther.
M = 119 g F = 176-177 °C Rdt = 87 %

1.5. 6-bromométhyl-2-[2-[2-(triphénylméthyl)-1*H*-tétrazol-5-yl]phényl]quinoléine

Dans 300 ml de tétrachlorure de carbone on ajoute 10 g (0,189 mole) du composé précédemment obtenu en 1.4 et on porte le mélange vers 60 °C jusqu'à dissolution totale. A cette température, on ajoute en une fois 3,7 g (0,208 mole) de N-bromosuccinimide et 60 mg (0,0037 mole) d'α,α'-azobisisobutyronitrile. On porte le mélange à température de reflux pendant 2 à 3 heures, jusqu'à disparition du N-bromosuccinimide. Au mélange refroidi, on ajoute 100 ml d'eau et 300 ml de dichlorométhane. On lave la phase organique plusieurs fois à l'eau, puis on la sèche. On évapore le solvant et on triture le résidu dans du diisopropyléther. On obtient un produit pur à 90 % qu'on utilisera tel quel.
M = 10,3 g

1.6. 2-butyl-4-chloro-1-[[2-[2-[2-(triphénylméthyl)-1*H*-tétrazol-5-yl]phényl]quinoléin-6-yl]méthyl]-1*H*-imidazole-5-carboxaldéhyde

a) 2-butyl-5-chloro-imidazol-4-méthanol
A 10 g (0,065 mole) de 2-butyl-imidazol-4-méthanol en suspension dans 200 ml d'acétate d'éthyle on introduit, à une température comprise entre 0 et 5 °C, 9,52 g (0,071 mole) de N-chlorosuccinimide. Tout en maintenant cette température, on agite le mélange pendant une nuit. On filtre la solution, on lave le solide avec 20 ml d'acétate d'éthyle glacé, on l'essore, on le lave à l'eau pour éliminer les traces de succinimide et de produit de départ puis on le sèche.
M = 7,3 g F = 143 °C Rdt = 73 %

b) 2-butyl-4-chloroimidazole-5-carboxaldéhyde
On ajoute goutte à goutte, de telle sorte que la température du milieu soit maintenue entre 22 et 28 °C, une solution renfermant 17,3 g (0,092 mole) du composé précédemment obtenu, dissous dans 52 ml d'acide acétique, à 133 g (0,243 mole) de nitrate de cérium et d'ammonium solubilisés dans 200 ml d'eau. On abandonne le milieu réactionnel pendant 3 à 5 heures jusqu'à ce que la solution devienne incolore. On refroidit le milieu et on amène le pH à 5-6 par addition de soude 10 N. On extrait le produit formé par 3 fractions d'éther, on lave la phase organique à l'eau bicarbonatée, on la sèche et on évapore le solvant. On obtient 16,4 g de composé qu'on recristallise dans le cyclohexane.
M = 13,9 g F = 92-93 °C Rdt = 81 %

c) 2-butyl-4-chloro-1-[[2-[2-[2-(triphénylméthyl)-1*H*-tétrazol-5-yl]phényl]quinoléin-6-yl]méthyl]-1*H*-imidazole-5-carboxaldéhyde
A 5,07 g (0,027 mole) de 2-butyl-4-chloroimidazole-5-carboxaldéhyde en solution dans 40 ml de diméthylformamide, on ajoute, en refroidissant au bain de glace, 5,11 g (0,037 mole) de carbonate de potassium et par portions 19,1 g (0,028 mole) du composé obtenu précédemment en 1.5. On agite le mélange sous argon pendant une nuit en laissant la température revenir à l'ambiante. On verse le milieu réactionnel dans l'eau, on recueille le solide formé et on le sèche. On purifie le composé obtenu par passage sur colonne de silice en éluant avec un mélange toluène/acétate d'éthyle (90/10).
M = 11,9 g F = 165 °C Rdt = 57%

1.7. 2-butyl-4-chloro-1-[[2-[2-(1*H*-tétrazol-5-yl)phényl]quinoléin-6-yl]méthyl]-1*H*-imidazole-5-carboxaldéhyde, chlorhydrate

A 11 g (0,015 mole) du composé obtenu précédemment en 1.6, en solution dans 130 ml de tétrahydrofurane on ajoute 10 ml (0,040 mole) d'acide chlorhydrique 4 N. On agite le mélange sous atmosphère d'argon pendant une nuit à température ambiante.
Le chlorhydrate du produit attendu cristallise dans le milieu réactionnel. On le recueille par filtration et on le lave avec 10 ml de tétrahydrofurane glacé.
M = 7,4 g F = 185 °C (déc) Rdt = 94%
RMN $^1H$ (200 MHz, DMSO): δ 0,8 (t, 3H), 1,2-1,4 (m, 2H), 1,5-1,65 (m, 2H), 5,8 (s, 2H), 7,6-8,0 (m, 8h), 8,6 (d, 1H), 9,7 (s,1H)

Exemple 2

Acide 2-butyl-4-chloro-1-[[2-[2-(1*H*-tétrazol-5-yl)phényl]-quinoléin-6-yl]méthyl]-1*H*-imidazole-5-carboxylique

A 344 mg (0,73 mmole) du dérivé aldéhydique obtenu précédemment en 1.6, on ajoute 5 ml de méthanol, 241 mg (3,7 mmoles) de cyanure de potassium, 64 µl d'acide acétique et 1,5 g de dioxyde de manganèse. On agite le mélange pendant 48 heures à la température ambiante. On recueille l'ester méthylique formé après filtration et évaporation. On ajoute immédiatement 2,55 ml (2,55 mmoles) de soude 1 N et on abandonne la solution pendant 3 heures à la température ambiante. On ajuste le pH de la solution à 3,5, on fitre l'insoluble, on le lave et on le sèche.
M = 230 mg F = 170 °C (déc) Rdt = 70%
RMN [1] (200 MHz, DMSO): 0,8 (t, 3H), 1,2-1,4 (m, 2H), 1,5-1,65 (m, 2H), 2,65 (t, 2H), 5,8 (s, 2H), 7,45-8 (m, 8H), 8,6 (d, 1H)

Exemple 3

2-butyl-4-chloro-1-[[2-[2-(1*H*-tétrazol-5-yl)phényl]quinoléin-6-yl]méthyl]-1*H*-imidazole-5-méthanol

3.1. 2-butyl-4-chloro-1-[[2-[2-[2-(triphénylméthyl)-1*H*-tétrazol-5-yl]phényl]quinoléin-6-yl]méthyl]-1*H*-imidazole-5-méthanol

A 1,65 g (2,3 mmoles) de composé obtenu en 1.6, en solution dans 150 ml de méthanol, on ajoute par petites portions, 330 mg (6,9 mmoles) de borohydrure de sodium . Après 30 minutes de réaction, on concentre le mélange et on le verse dans une solution de soude 2 N. On extrait le composé obtenu par du dichlorométhane. On purifie le produit brut ainsi obtenu par chromatographie sur colonne de silice avec un mélange chloroforme/acétate d'éthyle (80/20).
M = 1,12 g F = 175 °C Rdt = 68%

3.2. 2-butyl-4-chloro-1-[[2-[2-(1*H*-tétrazol-5-yl)phényl]quinoléin-6-yl]méthyl]-1*H*-imidazole-5-méthanol

On met en solution 1,1 g du composé obtenu précédemment en 3.1 dans un mélange contenant 31 ml de tétrahydrofurane, 31 ml de méthanol et 5 ml d'acide acétique. On porte la solution au reflux pendant 24 heures. On évapore les solvants, on triture le résidu dans l'éther et on recueille l'insoluble par filtration. On recristallise le produit dans la 2-butanone.
M = 450 mg F = 150-152 °C Rdt = 62%
RMN $^1$H (400MHz, DMSO): δ 0,75 (t, 3H), 1,25 (m, 2H), 1,5 (m, 2H), 2,53 (m, 2H), 4,36 (s, 2H), 5,2 (s, 1H), 5,46 (s, 2H), 7,53, (m, 3H), 7,7-7,79 (m, 4H), 7,93 (d, 1H), 8,3 (d, 1H).

Exemple 4

2-butyl-4-phénéthyl-1-[[2-[2-(1*H*-tétrazol-5-yl)phényl]quinoléin-6-yl]méthyl]-1*H*-imidazole-5-carboxaldéhyde

4.1. 2-butyl-4-phényléthénylimidazole-5-carboxaldéhyde

Sous atmosphère d'argon, dans 80 ml de toluène sec, on ajoute à 6 g de 2-n-butyl-4-iodoimidazole-5-carboxaldéhyde, 10,2 g de (*E*)-β-tri-n-butylstannylstyrène et 1 g de tétrakis(triphénylphosphine)palladium(0). On porte le mélange au reflux pendant 6 heures. On clarifie la solution par filtration après addition de noir animal et on évapore le solvant. On reprend le résidu par de l'hexane pour éliminer les dérivés d'étain. On purifie le composé obtenu sous forme de chlorhydrate ou d'oxalate.
chlorhydrate F = 225 °C (décomposition)
oxalate F = 217 °C
M = 5,1 g Rdt = 99%

4.2. 2-butyl-4-phénéthylimidazole-5-carboxaldéhyde

En présence de palladium sur charbon comme catalyseur, on soumet à une hydrogènation catalytique, à la température ambiante et à la pression atmosphérique, une solution de 2,5 g du composé obtenu précédemment en 4.1. et dissous dans 50 ml d'éthanol. Au bout de 30 minutes, on élimine le catalyseur par

filtration et on évapore le solvant. On obtient 2,5 g de composé attendu, sous forme de gomme. On purifie le composé obtenu sous forme de chlorhydrate.
chlorhydrate F = 179,5 °C

4.3. 2-butyl-4-phénéthyl-1-[[2-[2-[2-(triphénylméthyl)-1*H*-tétrazol-5-yl]phényl]quinoléin-6-yl]méthyl]-1*H*-imidazole-5-carboxaldéhyde

On obtient ce composé par réaction entre le 2-butyl-4-phénéthylimidazole-5-carboxaldéhyde et le dérivé bromé décrit dans l'exemple 1.5, selon le procédé décrit dans l'exemple 1.6.
F = 142,5 °C

4.4. 2-butyl-4-phénéthyl-1-[[2-[2-(1*H*-tétrazol-5-yl)phényl]quinoléin-6-yl]méthyl]-1*H*-imidazole-5-carboxaldéhyde

On obtient ce composé par détritylation du composé obtenu précédemment en 4.3 en chauffant 24 heures, dans du méthanol, à la température de reflux .
RMN $^1H$ (200MHz, $CDCl_3$): σ 0,8 (t, 3H), 1,25 (m, 2H), 1,6 (m, 2H), 2,5 (t, 2H), 3,1 (m, 4H), 5,75 (s, 2H), 7-7,5 (m, 11H), 7,6 (d, 1H), 7,75 (d, 1H), 7,9 (d, 1H), 9,55 (s, 1H).

Exemple 5

2-butyl-4-phénéthyl-1-[[2-[2-(1*H*-tétrazol-5-yl)phényl]quinoléin-6-yl]méthyl]-1*H*-imidazole-5-méthanol

5.1. 2-butyl-4-phénéthyl-1-[[2-[2-[2-(triphénylméthyl)-1*H*-tétrazol-5-yl]phényl]quinoléin-6-yl]méthyl]-1*H*-imidazole-5-méthanol

On prépare ce composé à partir du composé obtenu précédemment en 4.3, selon le procédé décrit dans l'exemple 3.1.
F = 150 °C

5.2. 2-butyl-4-phénéthyl-1-[[2-[2-(1*H*-tétrazol-5-yl)phényl]quinoléin-6-yl]méthyl]-1*H*-imidazole-5-méthanol

On prépare ce composé par détritylation du composé obtenu précédemment en 5.1, en chauffant 24 heures, dans du méthanol, à la température de reflux.
RMN $^1H$ (200MHz, $CDCl_3$-DMSO): σ 0,8 (t, 3H), 1,3 (m, 2H), 1,6 (m, 2H), 2,65 (t, 2H), 2,9 (m, 4H), 4,15 (s, 2H), 5,4 (s, 2H), 7,15-7,9 (m, 13H), 8,1 (d, 1H).

Exemple 6

6-[[2-butyl-4-chloro-5-(méthoxyméthyl)-1*H*-imidazol-1-yl]méthyl]-2-[2-(1*H*-tétrazol-5-yl)phényl]quinoléine

Dans 36 ml de méthanol, on introduit 1,5 g du composé décrit dans l'exemple 3 et on ajoute 0,18 ml d'acide sulfurique concentré. On chauffe à la température de reflux pendant 15 heures, on évapore le méthanol et on reprend le résidu par un mélange d'hydroxyde de sodium 1 N et de toluène. On recueille la phase aqueuse et on l'acidfie à pH 3 avec de l'acide chlorhydrique. On filtre le précipité formé et on le purifie par chromatographie sur colonne de gel de silice en éluant par un mélange dichlorométhane/méthanol/acide acétique (95/5/0,1).
M = 0,4 g F = 102 °C (déc) Rendement = 40 %

Le tableau suivant illustre les structures et les propriétés physiques de quelques composés selon l'invention.

Tableau

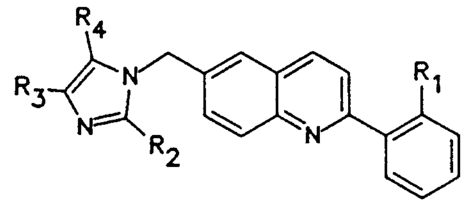

(I)

| No | $R_1$ | $R_2$ | $R_3$ | $R_4$ | Sel | F (°C) |
|---|---|---|---|---|---|---|
| 1 | $-CN_4H$ | $-(CH_2)_3CH_3$ | -Cl | -CHO | HCl | 185 (dec)* |
| 2 | $-CN_4H$ | $-(CH_2)_3CH_3$ | -Cl | $-CH_2OH$ | - | 150-152* |
| 3 | $-CN_4H$ | $-(CH_2)_3CH_3$ | -Cl | -COOH | - | 170 (dec) |
| 4 | $-CN_4H$ | $-(CH_2)_3CH_3$ | phénéthyle | -CHO | - | -* |
| 5 | $-CN_4H$ | $-(CH_2)_3CH_3$ | phénéthyle | $-CH_2OH$ | - | 184 (dec*) |
| 6 | $-CN_4H$ | $-(CH_2)_3CH_3$ | -Cl | -CN | - | 138 (dec) |
| 7 | $-CN_4H$ | $-(CH_2)_3CH_3$ | -Cl | $-CH_2OCH_3$ | - | 98 (dec) |
| 8 | $-CN_4H$ | $-(CH_2)_3CH_3$ | -Cl | $-COOCH_3$ | - | 181-184 |
| 9 | $-CN_4H$ | $-(CH_2)_3CH_3$ | -Cl | $-CH_2OC_2H_5$ | - | 170-172,5 |

| No | $R_1$ | $R_2$ | $R_3$ | $R_4$ | Sel | F (°C) |
|---|---|---|---|---|---|---|
| 10 | $-CN_4H$ | $-(CH_2)_3CH_3$ | -Cl | -CHO | - | 186 (dec) |
| 11 | $-CN_4H$ | $-(CH_2)_3CH_3$ | -Cl | $-CH_2O(CH_3)_2$ | - | 157,5-160 |
| 12 | $-CN_4H$ | $-(CH_2)_3CH_3$ | -Cl | $-CH_2O(CH_2)_3CH_3$ | - | 91 (dec) |
| 13 | $-CN_4H$ | $-(CH_2)_3CH_3$ | -Cl | $-CH_2OCH_2CH(CH_3)_2$ | - | 90 (dec) |
| 1 | $-CN_4H$ | $-(CH_2)_3CH_3$ | -Cl | $-CH_2OCH(CH_3)CH_2CH_3$ | | 98 (dec) |
| 15 | $-CN_4H$ | $-(CH_2)_3CH_3$ | -Cl | $-CH_2O(CH_2)_2OCH_3$ | - | 119,5-122,5 |
| 16 | $-CN_4H$ | $-(CH_2)_3CH_3$ | -Cl | $-CH_2O(CH_2)_2CH_3$ | - | 139-140 |
| 17 | $-CN_4H$ | $-(CH_2)_3CH_3$ | -Cl | $-CH_2OCH_2\underline{C}C_3H_7$ | - | 83 (dec) |
| 18 | $-CN_4H$ | $-(CH_2)_4CH_3$ | -Cl | -CHO | HCl | 188 (dec) |
| 19 | $-CN_4H$ | $-(CH_2)_4CH_3$ | -Cl | $-CH_2OH$ | - | 160-163 |
| 20 | $-CN_4H$ | $-CH_2CH_3$ | -Cl | -CHO | - | 197-199 |
| 21 | $-CN_4H$ | $-(CH_2)_4CH_3$ | -Cl | $-COOCH_2CH_3$ | - | 142-144 |
| 22 | $-CN_4H$ | $-(CH_2)_2CH_3$ | $-C_2H_5$ | $-COOCH_2CH_3$ | - | 196 (dec) |
| 23 | $-CN_4H$ | $-(CH_2)_3CH_2$ | -Cl | $-COOCH_2CH_3$ | - | 151-152 |
| 24 | $-CN_4H$ | $-CH_2CH_3$ | -Cl | $-COOCH_2CH_3$ | - | 188-189 |
| 25 | $-CN_4H$ | $-(CH_2)_4CH_3$ | -Cl | $-CH_2OCH_3$ | - | 106-108 |

| No | $R_1$ | $R_2$ | $R_3$ | $R_4$ | Sel | F (°C) |
|---|---|---|---|---|---|---|
| 26 | $-CN_4H$ | $-CH_2CH_3$ | -Cl | -COOH | - | 250 |
| 27 | $-CN_4H$ | $-(CH_2)_2CH_3$ | $-C_2H_5$ | -COOH | $H_2O$ | 177 |
| 28 | $-CN_4H$ | $-(CH_2)_2-CH_3$ | -Cl | -CHO | - | 180,5 |
| 29 | $-CN_4H$ | $-(CH_2)_2-CH_3$ | -Cl | $-CH_2OH$ | - | 166 (dec) |

Légende du tableau

HCl signifie chlorhydrate

dec. signifie décomposition

$CN_4H$ représente le groupe 1*H*-tétrazol-5-yle

$\underline{c}C_3H_7$ représente le groupe cyclopropyle

* produit caractérisé par spectre RMN (voir exemple correspondant)

Les composés de l'invention ont fait l'objet d'études pharmacologiques qui ont mis en évidence leurs propriétés antagonistes de l'angiotensine II.

Essai de liaison (binding) de [$^3$H]-angiotensine II sur la corticosurrénale de lapin.

On utilise des lapins mâles Fauves de Bourgogne de 2 à 3 kg. Après sacrifice par dislocation cervicale, on excise les glandes surrénales et on dissèque le cortex sur une boite de culture refroidie par de la glace. On le place dans 10 ml d'une solution tampon glacée à 10 mM de tris(hydroxyméthyl)aminométhane, contenant 0,33 M de saccharose et 1 mM d'acide éthylènediaminetétraacétique et dont le pH a été ajusté à 7,4 avec de l'acide chlorhydrique. On homogénéise le tissu à l'aide d'un appareil Potter électrique par 13 allers et retours de piston à la vitesse de 1200 tours par minute. On ajuste le volume de la préparation à 25 ml avec du tampon Tris-saccharose avant de centrifuger 15 min à 1075 x g. Le surnageant est conservé. Le culot est à nouveau homogénéisé, après remise en suspension dans 10 ml de tampon Tris-saccharose, par passage au Potter électrique puis centrifugé dans les conditions décrites précédemment. Le surnageant obtenu est ajouté au premier, et ils sont centrifugés 30 min à 47 800 x g. On reprend finalement les culots par 150 volumes (soit 100 mg de tissu dans 15 ml de tampon) d'une solution tampon de Tris-HCl à 50 mM, contenant 150 mM de NaCl, 5 mM d'acide éthylènediaminetétraacétique, 1,25 µg/ml de bacitracine, 100 µM de fluorure de phényl-méthylsulfonyle et 0,2 % de sérum albumine bovine (pH = 7,4 à 25 °C).
Cette suspension contient les microsomes de corticosurrénales et sera utilisée telle quelle dans les études décrites ci-dessous.
On fait incuber des fractions aliquotes de 100 µl de suspension, en présence de [$^3$H]-angiotensine II (New England Nuclear, d'une activité spécifique de 61 Ci/mmole) dans un volume final de 1 ml de tampon Tris-HCl dont la composition a été précédemment décrite. Après 30 minutes d'incubation à 25 °C, on récupère les microsomes par filtration sur filtres de nitrate de cellulose Millipore HAWP™ 0,45 µm préalablement conditionnés par trempage dans une solution à 1 % de sérum albumine bovine. Les filtres sont lavés trois fois avec 5 ml de tampon Tris-HCl glacé. La quantité de radioactivité liée au tissu et retenue sur les filtres est mesurée par spectrométrie de scintillation.

La liaison non spécifique de la [$^3$H]-angiotensine II est mesurée par incubation en présence de 1 µM d'angiotensine II non radioactive. Cette liaison non spécifique représente 5 à 10 % de la quantité totale de radioactivité fixée sur le filtre. La liaison spécifique est la différence entre la radioactivité totale recueillie sur le filtre et la radioactivité non spécifique. On mesure la liaison de la [$^3$H]-angiotensine en présence de différentes concentrations de composés à étudier et on détermine graphiquement la $CI_{50}$, concentration du composé étudié qui inhibe 50 % de la liaison spécifique de la [$^3$H]-angiotensine II.
Les $CI_{50}$ des composés de l'invention se situent entre 5 nM et 10 µM.

Inhibition de la réponse à l'angiotensine II sur la pression artérielle de rat.

On utilise des rats mâles (Sprague-Dawley, Charles River France) pesant 250 à 280 g, que l'on anesthésie au pentobarbital sodique (55 mg/kg i.p.) et que l'on maintient sous respiration artificielle (Respirateur Harvard™; fréquence de respiration de 70 ml par minute, volume d'air 1 ml par 100 g de poids corporel). On "spinalise" les animaux à l'aide d'une tige métallique, introduite par l'orbite de l'oeil droit et descendue le long de la colonne vertébrale. On sectionne les nerfs vagues droit et gauche (bivagotomie); on ligature l'artère carotide droite, l'artère carotide gauche étant cathétérisée afin de mesurer la pression artérielle à l'aide d'une cellule de pression (type Statham™ P23Db). On cathétérise une veine fémorale en vue de l'administration de divers composés.
On mesure les variations de pression artérielle moyenne induites par l'angiotensine administrée par voie intraveineuse à la dose de 0,5 µg/kg avant l'administration des composés de l'invention et celles induites par l'angiotensine administrée dans les mêmes conditions 5 minutes après l'administration par voie intraveineuse des composés de l'invention ou 30 minutes après leur administration par voie orale. Les composés de l'invention sont administrés à des doses allant de 0,01 à 100 mg/kg.
Le pourcentage d'inhibition de la réponse contrôle à l'angiotensine II est utilisé pour apprécier le potentiel antagoniste à l'angiotensine II des composés de l'invention.

Les composés de l'invention ou leurs sels appropriés peuvent être utilisés pour le traitement de diverses formes de pathologies hypertensives et d'insuffisances cardiaque, rénale ou pulmonaire ainsi que pour le traitement du glaucome.

Les composés de l'invention ou leurs sels appropriés peuvent également être utilisés en association avec d'autres substances à activité cardiovasculaire, telles que les diurétiques, les α-bloquants, les β-bloquants, les antagonistes calciques ou les inhibiteurs de l'enzyme de conversion de l'angiotensine I.

Les composés de l'invention ou leurs sels appropriés peuvent être présentés sous toutes formes pharmaceutiques appropriées au traitement pour l'administration orale, parentérale, intramusculaire ou rectale : comprimés, capsules, gélules, solutions ou suspensions stériles, suppositoires etc...

Pour le traitement du glaucome, les composés de l'invention peuvent être présentés sous la forme de comprimés, gélules, solutions injectables ou formulations oculaires topiques.

Les composés de l'invention peuvent être administrés aux patients en une quantité pouvant aller de 1 à 1000 mg par jour et par patient, en une ou plusieurs doses.

ANNEXE I

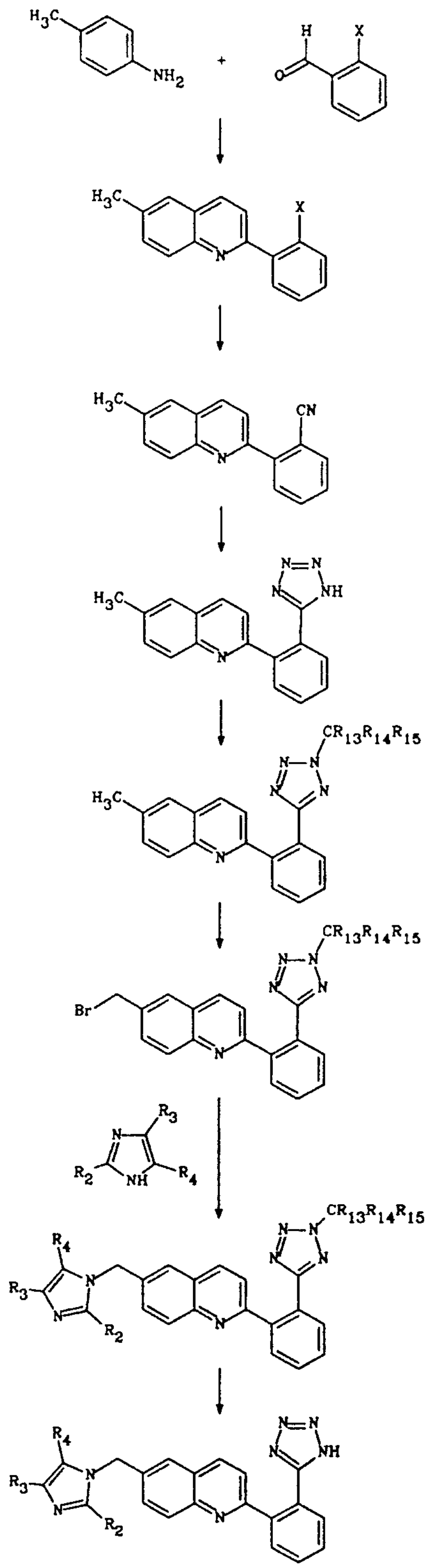

(II)

(III)

(IV)

(V)

(VI)

(VII)

(VIII)

(IX)

(I bis)

## ANNEXE II

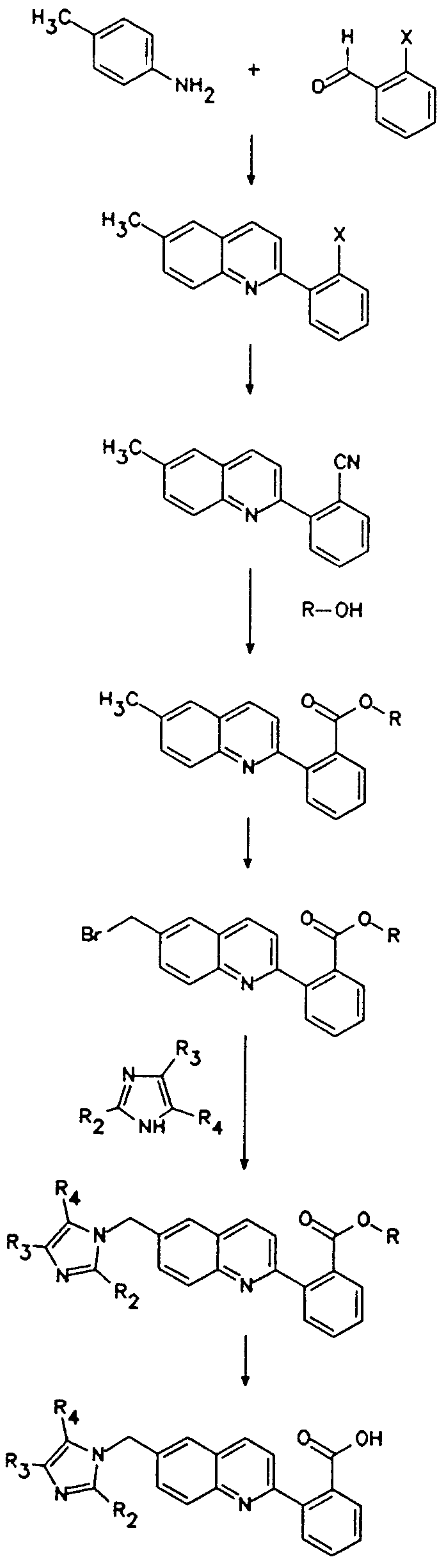


(II)

(III)

(IV)

(V')

(VI')

(VII')

(VIII)

(IX')

(I ter)

## Revendications

1. Dérivés de quinoléine répondant à la formule (I)

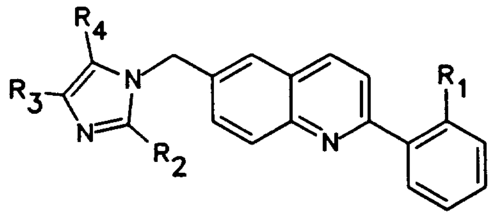

(I)

dans laquelle
$R_1$ représente soit un groupe 1*H*-tétrazol-5-yle, soit un groupe $CO_2H$,
$R_2$ représente soit un groupe $(C_{1-7})$alkyle, soit un groupe $(C_{2-6})$ alcényle,
$R_3$ et $R_4$ représentent, chacun indépendamment l'un de l'autre, soit un atome d'hydrogène, soit un atome d'halogène, soit un groupe cyano, soit un groupe $(C_{1-7})$alkyle, soit un groupe $(C_{3-7})$cycloalkyl-$(C_{1-4})$alkyle, soit un groupe aryle, soit un groupe aryl$(C_{1-4})$alkyle, soit un groupe aryl$(C_{2-6})$alcényle, soit un groupe $-(CH_2)_mCOR_5$ dans lequel m = 0 à 4 et $R_5$ représente un atome d'hydrogène, un groupe OH, un groupe $(C_{1-6})$alcoxy ou un groupe $NR_7R_8$, $R_7$ et $R_8$, représentant, chacun indépendamment l'un de l'autre, un atome d'hydrogène ou un groupe $(C_{1-4})$alkyle, soit un groupe $-(CH_2)_n-R_6$ dans lequel n = 1 à 4 et $R_6$ représente un groupe OH, un groupe $(C_{1-6})$alcoxy, un groupe $(C_{1-4})$alcoxy-$(C_{1-4})$alcoxy ou un groupe $(C_{3-7})$cycloalkyl$(C_{1-4})$alcoxy,
ainsi que leurs sels d'addition aux acides pharmaceutiquement acceptables.

2. Dérivés selon la revendication 1, caractérisés en ce que $R_1$ représente soit un groupe 1*H*-tétrazol-5-yle, soit un groupe $CO_2H$,
$R_2$ représente un groupe $(C_{1-7})$alkyle,
$R_3$ représente soit un atome d'halogène, soit un groupe $(C_{1-7})$alkyle, soit un groupe aryl$(C_{1-4})$alkyle, et
$R_4$ représente soit un groupe $-(CH_2)_m-COR_5$ dans lequel m = 0 à 4 et $R_5$ représente un atome d'hydrogène, un groupe OH, un groupe $(C_{1-6})$alcoxy ou un groupe $NR_7R_8$, $R_7$ et $R_8$ représentant indépendamment l'un de l'autre, un atome d'hydrogène ou un groupe $(C_{1-4})$alkyle, soit un groupe $-(CH_2)_n-R_6$ dans lequel n = 1 à 4 et $R_6$ représente un groupe OH ou un groupe $(C_{1-6})$alcoxy.

3. Dérivés selon la revendication 2, caractérisés en ce que
$R_1$ représente un groupe 1*H*-tétrazol-5-yle,
$R_2$ représente un groupe butyle,
$R_3$ représente soit un atome de chlore, soit un groupe éthyle, soit un groupe phénéthyle, et
$R_4$ représente un groupe $CH_2OH$, CHO, $CO_2H$, $CO_2CH_3$, $CO_2C_2H_5$, $CH_2OCH_3$.

4. Le 2-butyl-4-chloro-1-[[2-[2-(1*H*-tétrazol-5-yl)phényl]quinoléin-6-yl]méthyl]-1*H*-imidazole-5-carboxaldéhyde ainsi que ses sels d'addition aux acides et aux bases pharmaceutiquement acceptables.

5. L'acide 2-butyl-4-chloro-1-[[2-[2-(1*H*-tétrazol-5-yl) phényl]quinoléin-6-yl]méthyl]-1*H*-imidazole-5-carboxylique ainsi que ses sels d'addition aux acides et aux bases pharmaceutiquement acceptables.

6. Le 2-butyl-4-chloro-1-[[2-[2-(1*H*-tétrazol-5-yl)phényl] quinoléin-6-yl)méthyl]-1*H*-imidazole-5-méthanol ainsi que ses sels d'addition aux acides et aux bases pharmaceutiquement acceptables.

7. La 6-[[2-butyl-4-chloro-5-(méthoxyméthyl)-1*H*-imidazol-1-yl]méthyl]-2-[2-(1*H*-tétrazol-5-yl)phényl]-quinoléine ainsi que ses sels d'addition aux acides et aux bases pharmaceutiquement acceptables.

8. L'acide 2-propyl-4-éthyl-1-[[2-[2-(1*H*-tétrazol-5-yl)phényl]quinoléin-6-yl]méthyl]-1*H*-imidazole-5-carboxylique ainsi que ses sels d'addition aux acides et aux bases pharmaceutiquement acceptables.

**9.** Le 2-propyl-4-éthyl-1-[[2-[2-(1*H*-tétrazol-5-yl)phényl)quinoléin-6-yl]méthyl]-1*H*-imidazole-5-carboxylate d'éthyle ainsi que ses sels d'addition aux acides et aux bases pharmaceutiquement acceptables.

**10.** Le 2-butyl-4-chloro-1-[[2-[2-(1*H*-tétrazol-5-yl)phényl] quinoléin-6-yl]méthyl]-1*H*-imidazole-5-carboxylate d'éthyle ainsi que ses sels d'addition aux acides et aux bases pharmaceutiquement acceptables.

**11.** Procédé de préparation des composés selon la revendication 1, procédé caractérisé en ce que l'on fait réagir la 4-méthylbenzènamine avec un benzaldéhyde de formule (II)

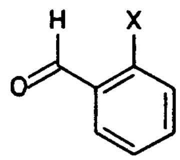

(II)

dans laquelle X représente un atome de brome ou d'iode en présence d'acide propiolique, ensuite on fait réagir le composé (III) obtenu

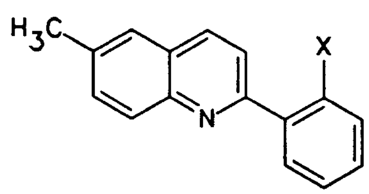

(III)

avec du cyanure cuivreux, puis . soit on fait réagir le 2-(6-méthylquinoléin-2-yl)benzonitrile (IV)

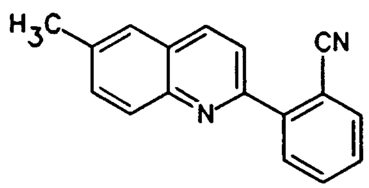

(IV)

avec un azidure organométallique ou métallique, puis on fait passer sur le composé obtenu un courant d'acide chlorhydrique gazeux, ensuite on protège le groupe tétrazole de la 6-méthyl-2-[2-(1*H*-tétrazol-5-yl)phènyl]quinoléine (V)

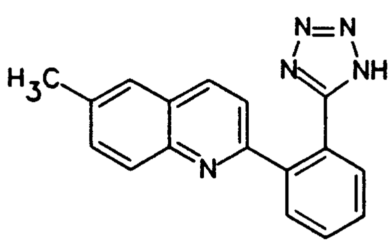

(V)

par un groupe protecteur de formule $CR_{13}R_{14}R_{15}$, dans laquelle $R_{13}$, $R_{14}$ et $R_{15}$ représentent chacun indépendamment l'un de l'autre un groupe $(C_{1-2})$alkyle ou un groupe aryle, et on fonctionnalise le groupe méthyle du composé de formule (VI)

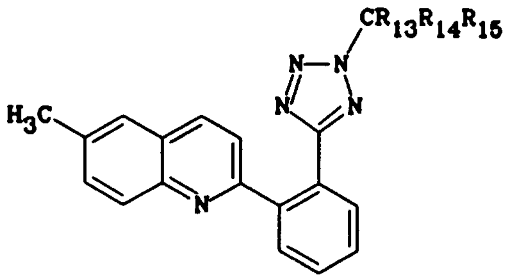

(VI)

en y introduisant un groupe partant, puis on fait réagir le composé (VII) obtenu

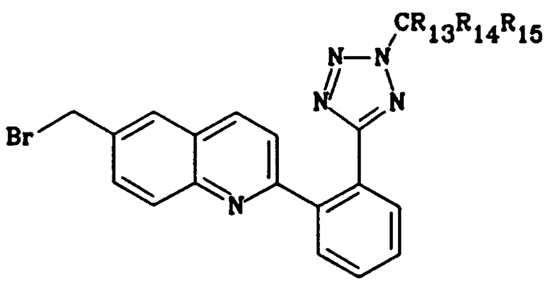

(VII)

avec un imidazole de formule (VIII)

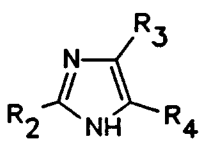

(VIII)

dans laquelle $R_2$, $R_3$ et $R_4$ sont tels que définis dans la revendication 1, et enfin on réalise la déprotection du groupe tétrazolyle du dérivé de formule (IX),

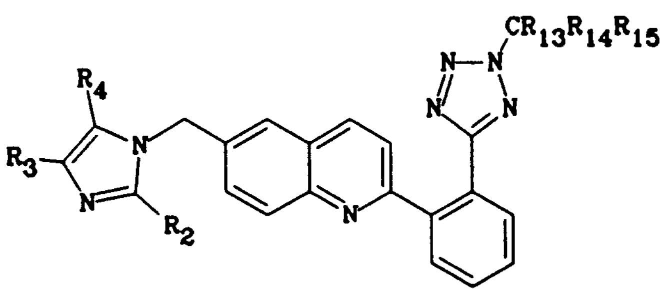

(IX)

de manière à obtenir un composé de formule (I bis)

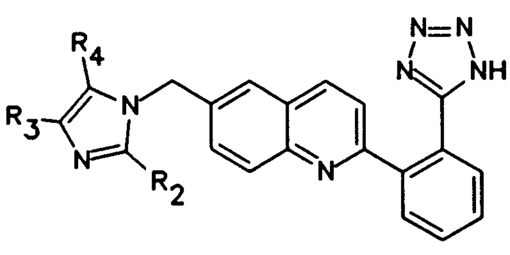

(I bis)

. soit on fait réagir, en milieu acide, le 2-(6-méthylquinoléin-2-yl)benzonitrile (IV)

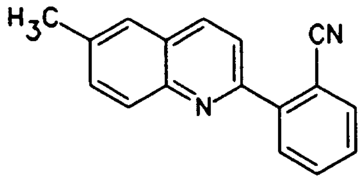

(IV)

avec un alcool de formule (V')

R-OH (V')

dans laquelle R est un radical $(C_{1-4})$alkyle ramifié ou non, puis on fonctionnalise le groupe méthyle en position 6 de la quinoléine de formule (VI')

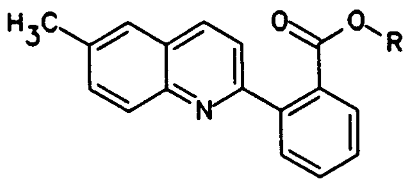

(VI')

en y introduisant un groupe partant et on fait réagir la quinoléine de formule (VII')

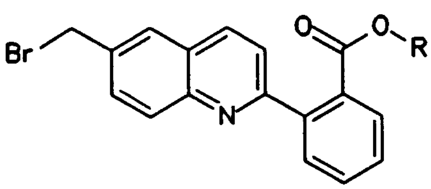

(VII')

avec un imidazole de formule (VIII)

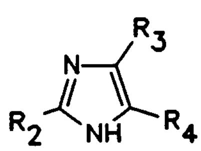

(VIII)

dans laquelle $R_2$, $R_3$ et $R_4$ sont tels que définis dans la revendication 1, puis on hydrolyse la fonction ester de la quinoléine de formule (IX')

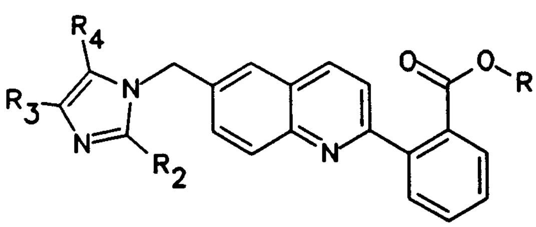

(IX')

de manière à obtenir un composé de formule (I ter)

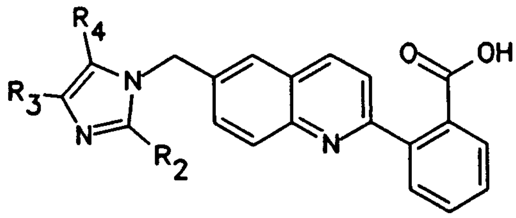

(I ter)

**12.** Médicament caractérisé en ce qu'il contient un composé selon l'une quelconque des revendications 1 à 10.

**13.** Composition pharmaceutique, caractérisée en ce qu'elle contient un composé selon l'une quelconque des revendications 1 à 10, en association avec tout excipient approprié.

**14.** Composés répondant à la formule (X)

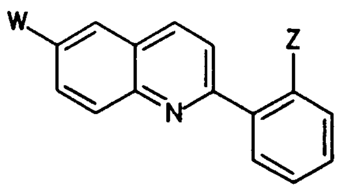

(X)

dans laquelle
W représente soit un groupe méthyle, soit un groupe $-CH_2R_{11}$ dans lequel $R_{11}$ représente un atome de chlore, un atome de brome ou un groupe partant $OR_{12}$, $R_{12}$ étant un groupe tel qu'un groupe tosyle ou un groupe mésyle,
Z représente soit un atome d'halogène, soit un groupe cyano, soit un groupe 1*H*-tétrazol-5-yle, soit un groupe 1*H*-tétrazol-5-yle protégé par un groupe protecteur de formule $CR_{13}R_{14}R_{15}$, dans laquelle $R_{13}$, $R_{14}$ et $R_{15}$ représentent chacun indépendamment l'un de l'autre un groupe $(C_{1-2})$alkyle ou un groupe aryle, soit un groupe COOR, R étant un groupe $(C_{1-4})$alkyle ramifié ou non utiles pour la synthèse des composés selon la revendication 1.

## Claims

**1.** Quinoline derivatives of the formula (I)

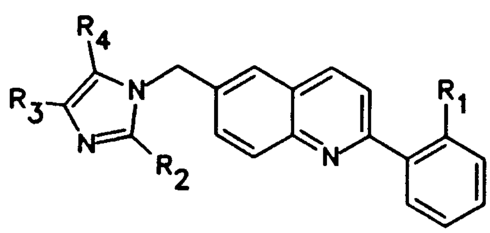

(I)

in which
$R_1$ represents either a 1*H*-tetrazol-5-yl group or a $CO_2H$ group,
$R_2$ represents either a $(C_{1-7})$alkyl group or a $(C_{2-6})$alkenyl group,
$R_3$ and $R_4$ represent, independently of each other, either a hydrogen atom or a halogen atom or a cyano group or a $(C_{1-7})$alkyl group or a $(C_{3-7})$cycloalkyl$(C_{1-4})$alkyl group or an aryl group or an aryl-$(C_{1-4})$alkyl group or an aryl$(C_{2-6})$alkenyl group or a $-(CH_2)_mCOR_5$ group in which m = 0 to 4 and $R_5$ represents a hydrogen atom, an OH group, a $(C_{1-6})$alkoxy group or an $NR_7R_8$, $R_7$ and $R_8$ representing, independently of each other, a hydrogen atom or a $(C_{1-4})$alkyl group or a $-(CH_2)_n-R_6$ group in which n

= 1 to 4 and $R_6$ represents an OH group, a $(C_{1-6})$ alkoxy group or a $(C_{1-4})$ alkoxy $(C_{1-4})$ alkoxy group or a $(C_{3-7})$cycloalkyl$(C_{1-4})$alkoxy group,
as well as their addition salts with pharmaceutically acceptable acids.

2. Derivatives according to claim 1, characterised in that
$R_1$ represents either a 1*H*-tetrazol-5-yl group or a $CO_2H$ group, $R_2$ represents a $(C_{1-7})$alkyl group,
$R_3$ represents either a halogen atom or a $(C_{1-7})$alkyl group or an aryl$(C_{1-4})$alkyl group, and
$R_4$ represents either a $-(CH_2)_mCOR_5$ group in which m = 0 to 4 and $R_5$ represents a hydrogen atom, an OH group, a $(C_{1-6})$alkoxy group or an $NR_7R_8$, $R_7$ and $R_8$ representing, independently of each other, a hydrogen atom or a $(C_{1-4})$alkyl group or a $-(CH_2)_n-R_6$ group in which n = 1 to 4 and $R_6$ represents an OH group, a $(C_{1-6})$alkoxy group.

3. Derivatives according to claim 2, characterized in that,
$R_1$ represents a 1*H*-tetrazol-5-yl group,
$R_2$ represents a butyl group,
$R_3$ represents either a chlorine atom or an ethyl group or a phenethyl group, and
$R_4$ represents a $CH_2OH$, CHO, $CO_2H$, $CO_2CH_3$, $CO_2C_2H_5$, $CH_2OCH_3$ group.

4. 2-butyl-4-chloro-1-[[2-[2-(1*H*-tetrazol-5-yl)phenyl]quinolin-6-yl]methyl]-1*H*-imidazole-5-carboxaldehyde as well as its addition salts with pharmaceutically acceptable acids and bases.

5. 2-butyl-4-chloro-1-[[2-[2-(1*H*-tetrazol-5-yl)phenyl] quinolin-6-yl]methyl]-1*H*-imidazole-5-carboxylic acid as well as its addition salts with pharmaceutically acceptable acids and bases.

6. 2-butyl-4-chloro-1-[[2-[2-(1*H*-tetrazol-5-yl)phenyl] quinolin-6-yl]methyl]-1*H*-imidazole-5-methanol as well as its addition salts with pharmaceutically acceptable acids and bases.

7. 6-[[2-butyl-4-chloro-5-(methoxymethyl)-1*H*-imidazol-1-yl] methyl]-2-[2-(1*H*-tetrazol-5-yl)phenyl]quinoline as well as its addition salts with pharmaceutically acceptable acids and bases.

8. 2-propyl-4-ethyl-1-[[2-[2-(1*H*-tetrazol-5-yl)phenyl] quinolin-6-yl]methyl]-1*H*-imidazole-5-carboxylic acid as well as its addition salts with pharmaceutically acceptable acids and bases.

9. Ethyl 2-propyl-4-ethyl-1-[[2-[2-(1*H*-tetrazol-5-yl)phenyl]quinolin-6-yl]methyl]-1*H*-imidazole-5-carboxylate as well as its addition salts with pharmaceutically acceptable acids and bases.

10. Ethyl 2-butyl-4-chloro-1-[[2-[2-(1*H*-tetrazol-5-yl)phenyl] quinolin-6-yl]methyl]-1*H*-imidazole-5-carboxylate as well as its addition salts with pharmaceutically acceptable acids and bases.

11. Process for preparing the compounds according to claim 1, which process is characterised in that 4-methylbenzenamine is reacted with a benzaldehyde of formula (II)

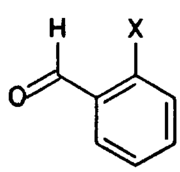

(II)

in which X represents a bromine or iodine atom, in the presence of propiolic acid, then the compound (III) obtained

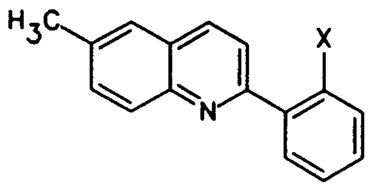

(III)

is reacted with copper(I) cyanide, and then . either 2-(6-methylquinolin-2-yl)benzonitrile (IV)

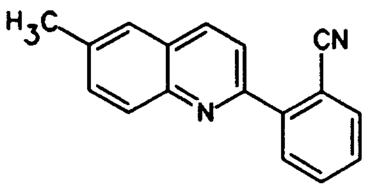

(IV)

is reacted with an organometallic or metallic azide, then a stream of gaseous hydrochloric acid is passed over the compound obtained, and then the tetrazole group of 6-methyl-2-[2-(1*H*-tetrazol-5-yl)-phenyl]quinoline (V)

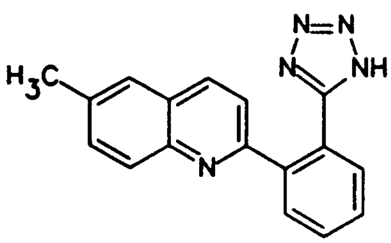

(V)

is protected with a protecting group of formula $CR_{13}R_{14}R_{15}$, in which $R_{13}$, $R_{14}$ et $R_{15}$ each represent independently of each other a $(C_{1-2})$alkyl group or an aryl group, and the methyl group of the compound of formula (VI)

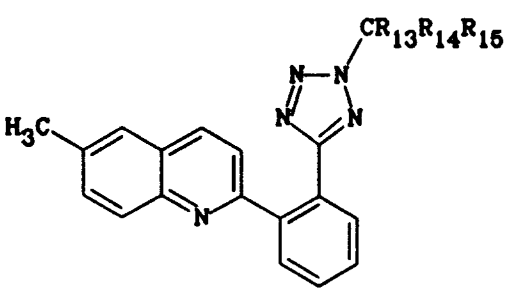

(VI)

is functionalised by introducing therein a departing group, and then the compound (VII) obtained

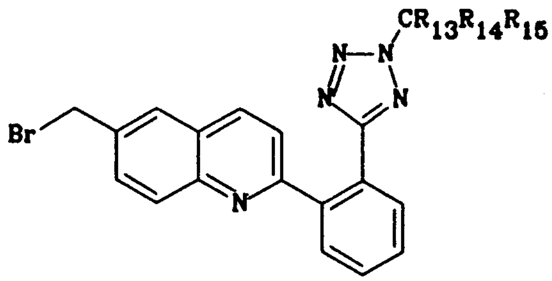

(VII)

is reacted with an imidazole of formula (VIII)

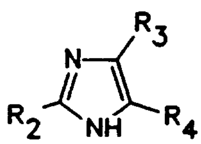

(VIII)

in which $R_2$, $R_3$ and $R_4$ are as defined in claim 1, and, finally, the deprotection of the tetrazolyl group of the derivative of formula (IX),

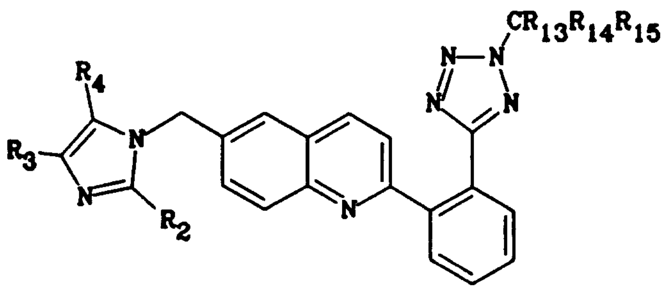

(IX)

is carried out in order to obtain a compound of formula (Ia)

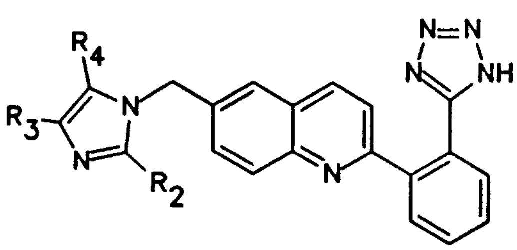

(Ia)

. or the 2-(6-methylquinolein-2-yl)benzonitrile (IV)

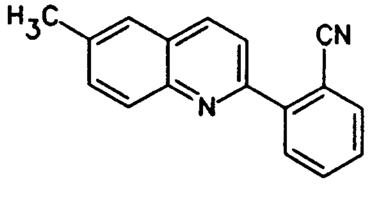

(IV)

is reacted in acidic medium with an alcohol of formula (V')

R-OH (V')

in which R is a branched or unbranched ($C_{1-4}$)alkyl radical, the methyl group in position 6 of the quinoline of formula (VI')

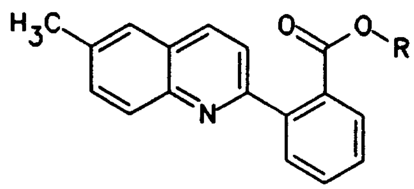

(VI')

is functionalised by introducing therein a departing group, and the quinoline of formula (VII')

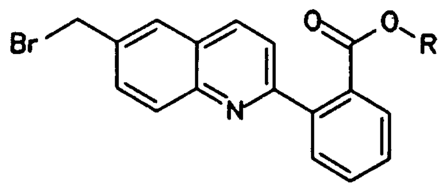

(VII')

is reacted with an imidazole of formula (VIII)

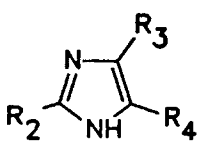

(VIII)

in which $R_2$, $R_3$ and $R_4$ are as defined in claim 1, and then the ester functional group of the quinoline of formula (IX')

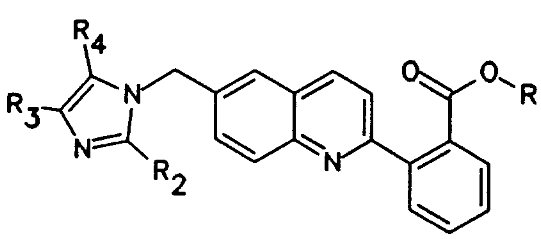

(IX')

is hydrolyzed in order to obtain a compound of formula (Ib)

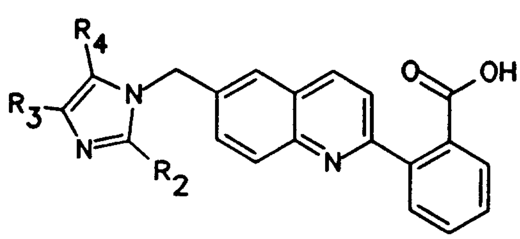

(Ib)

12. Medicinal product characterized in that it contains a compound according to any one of claims 1 to 10.

13. Pharmaceutical composition characterized in that it contains a compound according to any one of claims 1 to 10 in combination with any suitable excipient.

14. Compounds of the formula (X)

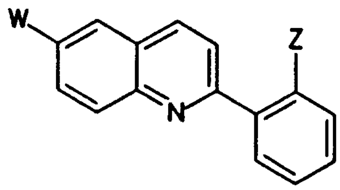

(X)

in which
W represents either a methyl group, or a $-CH_2R_{11}$ group in which
$R_{11}$ represents a chlorine atom, a bromine atom or a departing group $OR_{12}$, $R_{12}$ being a group such as a tosyl group or a mesyl group,
Z represents either a halogen atom or a cyano group or a 1*H*-tetrazol-5-yl group or a 1*H*-tetrazol-5-yl group protected by a protecting group of formula $CR_{13}R_{14}R_{15}$, $R_{13}$, $R_{14}$ and $R_{15}$ representing independently of each other a $(C_{1-2})$alkyl group or an aryl group, or a COOR, R being a branched or unbranched $(C_{1-4})$alkyl group,
which compounds are useful for the synthesis of the compounds according to claim 1.

**Patentansprüche**

1. Chinolinderivate der Formel (I)

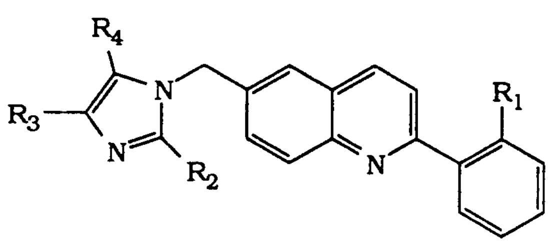

(I)

in der
$R_1$ entweder eine 1H-Tetrazol-5-yl-gruppe oder eine $CO_2H$-Gruppe,
$R_2$ entweder eine $(C_{1-7})$-Alkylgruppe oder eine $(C_{2-6})$-Alkenylgruppe,
$R_3$ und $R_4$ Jeweils unabhängig voneinander entweder ein Wasserstoffatom oder ein Halogenatom oder eine Cyanogruppe oder eine $(C_{1-7})$-Alkylgruppe oder eine $(C_{3-7})$-Cycloalkyl-$(C_{1-4})$-alkylgruppe, oder eine Arylgruppe, oder eine Aryl$(C_{1-4})$-alkylgruppe, oder eine Aryl-$(C_{2-6})$-alkenylgruppe oder eine Gruppe $-(CH_2)_m-COR_5$, in der m = 0 bis 4 und $R_5$ ein Wasserstoffatom, eine Hydroxylgruppe, eine $(C_{1-6})$-Alkoxygruppe oder eine Gruppe $NR_7R_8$ darstellen, worin $R_7$ und $R_8$ jeweils unabhängig voneinander ein Wasserstoffatom oder eine $(C_{1-4})$-Alkylgruppe darstellen, oder eine Gruppe $-(CH_2)_n-R_6$, in der n = 1 bis 4 und $R_6$ eine Hydroxylgruppe, eine $(C_{1-6})$-Alkoxygruppe, eine $(C_{1-4})$-Alkoxy-$(C_{1-4})$-alkoxygruppe oder eine $(C_{3-7})$-Cycloalkyl-$(C_{1-4})$-alkoxygruppe darstellen, bedeuten sowie deren Additionssalze mit pharmazeutisch annehmbaren Säuren.

2. Derivate nach Anspruch 1, **dadurch gekennzeichnet**, daß $R_1$ entweder eine 1H-Tetrazol-5-yl-gruppe oder eine $CO_2H$-Gruppe,
$R_2$ eine $(C_{1-7})$-Alkylgruppe,
$R_3$ entweder ein Halogenatom, oder eine $(C_{1-7})$-Alkylgruppe, oder eine Aryl$(C_{1-4})$-alkylgruppe und
$R_4$ entweder eine Gruppe $-(CH_2)_m-COR_5$, worin m = 0 bis 4 und $R_5$ ein Wasserstoffatom, eine

Hydroxylgruppe, eine $(C_{1-6})$-Alkoxygruppe oder eine $NR_7R_8$-Gruppe darstellen, worin $R_7$ und $R_8$ unabhängig voneinander ein Wasserstoffatom oder eine $(C_{1-4})$-Alkylgruppe darstellen, oder eine Gruppe $-(CH_2)_n-R_6$. worin n = 1 bis 4 und $R_6$ eine Hydroxylgruppe oder eine $(C_{1-6})$-Alkoxygruppe darstellen, bedeuten.

**3.** Derivate nach Anspruch 2, **dadurch gekennzeichnet**, daß $R_1$ eine 1H-Tetrazol-5-yl-gruppe,
$R_2$ eine Butylgruppe,
$R_3$ entweder ein Chloratom oder eine Ethylgruppe oder eine Phenethylgruppe und
$R_4$ eine Gruppe der Formel $CH_2OH$, CHO, $CO_2H$, $CO_2CH_3$, $CO_2C_2H_5$ oder $CH_2OCH_3$ bedeuten.

**4.** 2-Butyl-4-chlor-1-[[2-[2-(1H-tetrazol-5-yl)-phenyl]-chinolin-6-yl]-methyl]-1H-imidazol-5-carboxaldehyd sowie dessen Additionssalze mit pharmazeutisch annehmbaren Säuren und Basen.

**5.** 2-Butyl-4-chlor-1-[[2-[2-(1H-tetrazol-5-yl)-phenyl]-chinolin-6-yl]-methyl]-1H-imidazol-5-carbonsäure und deren Additionssalze mit pharmazeutisch annehmbaren Säuren und Basen.

**6.** 2-Butyl-4-chlor-1-[[2-[2-(1H-tetrazol-5-yl)-phenyl]-chinolin-6-yl]-methyl]-1H-imidazol-5-methanol sowie dessen Additionssalze mit pharmazeutisch annehmbaren Säuren und Basen.

**7.** 6-[[2-Butyl-4-chlor-5-(methoxymethyl)-1H-imidazol-1-yl]-methyl]-2-[2-(1H-tetrazol-5-yl)-phenyl]-chinolin sowie dessen Additionssalze mit pharmazeutisch annehmbaren Säuren und Basen.

**8.** 2-Propyl-4-ethyl-1-[[2-[2-(1H-tetrazol-5-yl)-phenyl]-chinolin-6-yl]-methyl]-1H-imidazol-5-carbonsäure sowie deren Additionssalze mit pharmazeutisch annehmbaren Säuren und Basen.

**9.** 2-Propyl-4-ethyl-1-[[2-[2-(1H-tetrazol-5-yl)-phenyl]-chinolin-6-yl]-methyl]-1H-imidazol-5-carbonsäureethylester und dessen Additionssalze mit pharmazeutisch annehmbaren Säuren und Basen.

**10.** 2-Butyl-4-chlor-1-[[2-[2-(1H-tetrazol-5-yl)-phenyl]-chinolin-6-yl]-methyl]-1H-imidazol-5-carbonsäureethylester sowie dessen Additionssalze mit pharmazeutisch annehmbaren Säuren und Basen.

**11.** Verfahren zur Herstellung der Verbindungen nach Anspruch 1, **dadurch gekennzeichnet**, daß man 4-Methylbenzolamin mit einem Benzaldehyd der Formel (II)

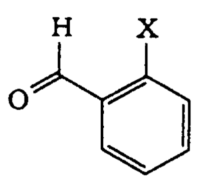

(II)

in der X ein Bromatom oder ein Iodatom bedeutet, in Gegenwart von Propiolsäure umsetzt und anschließend die erhaltene Verbindung (III)

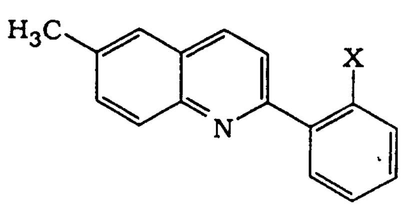

(III)

mit Kupfer(I)-cyanid umsetzt und dann . entweder das 2-(6-Methylchinolin-2-yl)-benzonitril (IV)

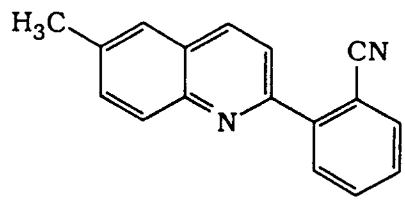

(IV)

mit einem metallorganischen Azid oder einem Metallazid umsetzt, dann einen Strom von gasförmiger Chlorwasserstoffsäure über die erhaltene Verbindung leitet, dann die Tetrazolgruppe des 6-Methyl-2-[2-(1H-tetrazol-5-yl)-phenyl]-chinolins (V)

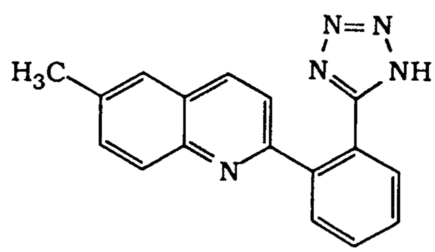

(V)

durch eine Schutzgruppe der Formel $CR_{13}R_{14}R_{15}$ schützt, in der $R_{13}$, $R_{14}$ und $R_{15}$ jeweils unabhängig voneinander eine $(C_{1-2})$-Alkylgruppe oder eine Arylgruppe bedeuten, und die Methylgruppe der Verbindung der Formel (VI)

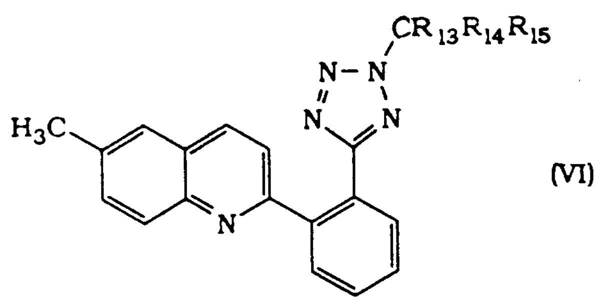

(VI)

funktionalisiert durch Einführen einer austretenden Gruppe, und dann die erhaltene Verbindung (VII)

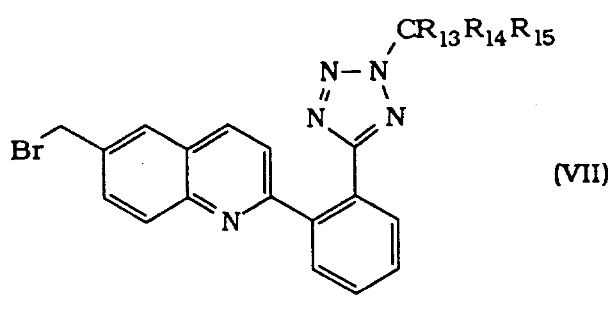

(VII)

mit einem Imidazol der Formel (VIII)

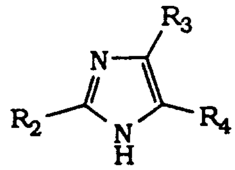

(VIII)

in der $R_2$, $R_3$ und $R_4$ die in Anspruch 1 angegebenen Bedeutungen besitzen, umsetzt und schließlich die Abspaltung der Schutzgruppen von der Tetrazolylgruppe des Derivats der Formel (IX)

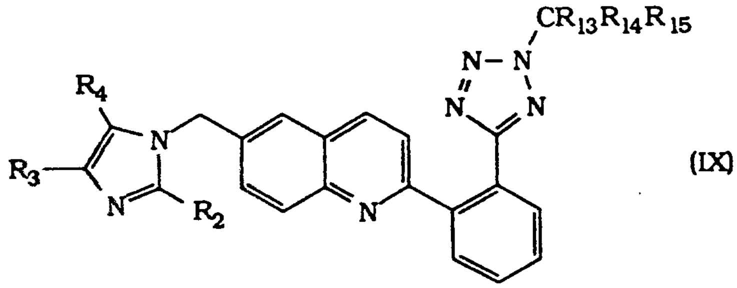

(IX)

bewirkt zur Bildung einer Verbindung der Formel (I bis)

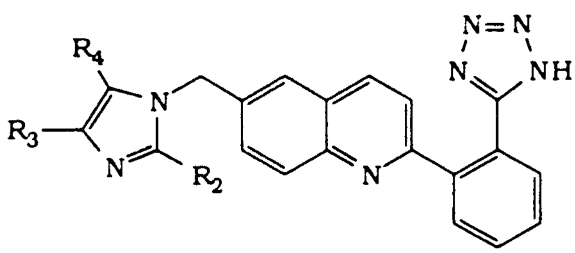

(I bis)

. oder das 2-(6-Methyl-chinolin-2-yl]-benzonitril (IV)

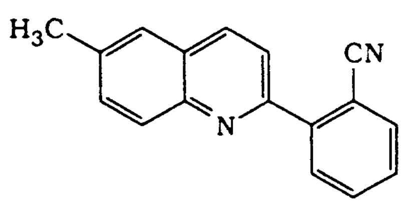

(IV)

in saurem Medium mit einem Alkohol der Formel (V')

R-OH (V')

in der R eine gegebenenfalls verzweigte ($C_{1-4}$)-Alkylgruppe darstellt, umsetzt und dann die Methylgruppe in der 6-Stellung des Chinolins der Formel (VI')

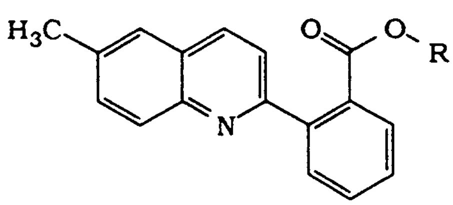

(VI')

funktionalisiert durch Einführen einer austretenden Gruppe und das Chinolin der Formel (VII')

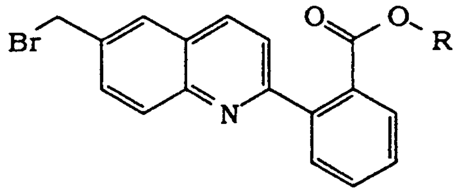

(VII')

mit einem Imidazol der Formel (VIII)

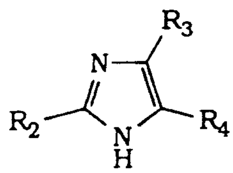

(VIII)

in der $R_2$, $R_3$ und $R_4$ die in Anspruch 1 angegebenen Bedeutungen besitzen, umsetzt und schließlich die Estergruppe des Chinolins der Formel (IX')

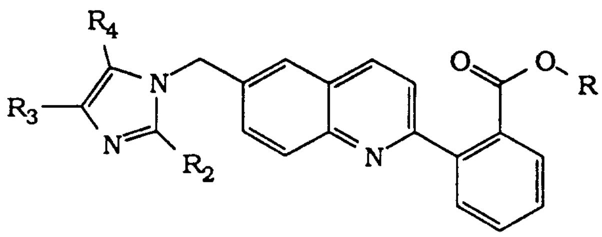

(IX')

hydrolysiert, so daß man eine Verbindung der Formel (I ter)

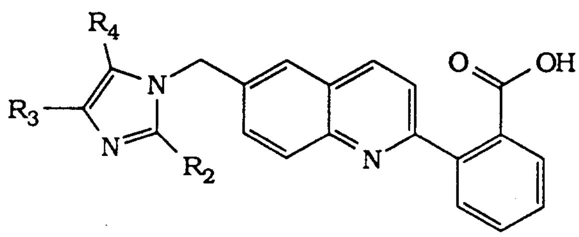

(I ter)

erhält.

**12.** Arzneimittel, **dadurch gekennzeichnet**, daß es eine Verbindung nach einem der Ansprüche 1 bis 10 enthalt.

**13.** Pharmazeutische Zubereitung, **dadurch gekennzeichnet**, daß sie eine Verbindung nach einem der Ansprüche 1 bis 10 in Kombination mit irgendeinem geeigneten Trägermaterial enthält.

**14.** Verbindungen der Formel (X)

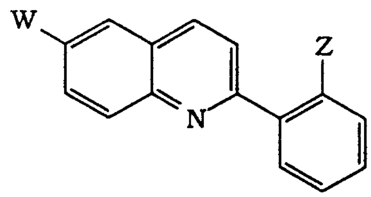

(X)

in der
W entweder eine Methylgruppe oder eine Gruppe $-CH_2R_{11}$, worin $R_{11}$ ein Chloratom, ein Bromatom oder eine austretende Gruppe $OR_{12}$ darstellt, worin $R_{12}$ eine Gruppe, wie eine Tosylgruppe oder eine Mesylgruppe bedeutet,
Z entweder ein Halogenatom oder eine Cyanogruppe oder eine 1H-Tetrazol-5-yl-gruppe oder eine 1H-Tetrazol-5-yl-gruppe, die durch eine Schutzgruppe der Formel $CR_{13}R_{14}R_{15}$ geschützt ist, in der $R_{13}$, $R_{14}$ und $R_{15}$ jeweils unabhängig voneinander eine $(C_{1-2})$-Alkylgruppe oder eine Arylgruppe darstellen, oder eine Gruppe COOR, worin R eine gegebenenfalls verzweigte $(C_{1-4})$-Alkylgruppe darstellt, bedeuten, für die Synthese der Verbindungen nach Anspruch 1.